# EUROPEAN PATENT APPLICATION

(11) **EP 4 047 013 A1**
(43) Date of publication of application: **24.08.2022**
(21) Application number: 20876195.7
(22) Date of filing: 25.09.2020
(51) Int. Cl.: C07K 14/34, C07K 14/22, C07K 14/285, C07K 14/21, C07K 19/00, C07K 1/10, A61K 39/09, A61K 39/385, A61P 31/04, A61P 11/00

(54) **CARRIER PROTEIN WITH SITE-DIRECTED MUTATION AND USE THEREOF IN PREPARATION OF VACCINE**

(30) Priority: 15.10.2019 CN 201910978406
(71) Applicant: Cansino Biologics Inc., Tianjin 300457 (CN)
(72) Inventor: WANG, Haomeng, Tianjin 300457 (CN); YAN, Qiaoling, Tianjin 300457 (CN); CHAO, Shoubai, Tianjin 300457 (CN); MAO, Huihua, Tianjin 300457 (CN); ZHU, Tao, Tianjin 300457 (CN)
(74) Representative: HGF
(86) International application number: PCT/CN2020/117825
(87) International publication number: WO 2021/073402

(57) **Abstract**

The present invention relates to a carrier protein with site-directed mutation and use thereof in preparation of a vaccine, wherein the carrier protein is selected from fusion proteins formed by one, two or more of diphtheria toxoid, a non-toxic mutant of diphtheria toxin, a bacterial outer membrane protein and a bacterially expressed protein, wherein an amino acid at at least one site on the carrier protein is mutated into an unnatural amino acid, and the unnatural amino acid contains an azido or alkynyl terminal group. In a mutual reaction process of the carrier protein with site-directed mutation of the present invention and a polysaccharide antigen, a covalent bond is formed, and meanwhile a formed conjugate is in a bead-string state, so that the carrier protein and the polysaccharide antigen can be effectively prevented from being excessively crosslinked. Further, particle size distribution of the conjugate is significantly uniform and controllable, which provides an effective means for improving quality of a polysaccharide-protein conjugate vaccine.

## Description

### TECHNICAL FIELD

The present invention relates to the field of biological pharmacy, and in particular to a carrier protein with site-directed mutation and use thereof in preparation of a vaccine, especially a multivalent conjugate vaccine for pneumonia.

### BACKGROUND

Infection by *Streptococcus pneumoniae* is one of the leading causes for morbidity and mortality worldwide. Pneumonia, febrile bacteremia and meningitis are the most common manifestations of invasive pneumococcal diseases, and dissemination of bacteria in the respiratory tract can lead to middle ear infection, nasosinusitis or recurrent bronchitis.

In polysaccharide-protein conjugate vaccines prepared by linking polysaccharide to a protein carrier, chemical bonds of the polysaccharide and the protein carrier can induce an immune response against bacteria that display on their surface the polysaccharide contained in the vaccine, thereby preventing diseases. Therefore, vaccination using polysaccharides from pathogenic bacteria is a potential strategy to enhance host immunity.

Although polysaccharides are immunogenic on their own, conjugation of polysaccharide to carrier protein has been used to improve immunogenicity. The carrier protein may be either a related protein antigen that comes from a target pathogen and enhances a specific immune response against that pathogen, or a generally immunogenic protein that primarily serves as an adjuvant or a general immune response stimulant.

A traditional polysaccharide-protein conjugation technology, such as a preparation method for polysaccharide-protein conjugate vaccine disclosed in Chinese patent ZL02159032.X, is also one of the most commonly used technologies for preparing polysaccharide conjugate vaccines at present. In the described technology, adipic acid dihydrazide (ADH) is used as a linker to conjugate the polysaccharide to the protein. With respect to this conjugation mode, firstly the polysaccharide needs to be activated by cyanogen bromide, and that is, cyanogen bromide is used for acting on hydroxyl group on polysaccharide molecule under an alkaline condition to form cyanate ester, and then the cyanate ester reacts with ADH; one carbon-oxygen bond in the cyanate ester is broken, and the cyanate ester is subjected to addition reaction with an amino group at one end of the ADH, so that ester hydrazide (AH) group is introduced into polysaccharide molecule to form polysaccharide-AH derivative; the polysaccharide-AH derivative forms a stable conjugate with the carrier protein under the mediation of carbodiimide (EDAC). Such conjugation mode can reduce the steric hindrance of the conjugation of the polysaccharide to the carrier protein, reserves the epitope of the polysaccharide, and meanwhile avoids the solubility of the polysaccharide and thus reduces the side effect of the reaction of the polysaccharide and the antiserum. However, this conjugation mode has the following deficiencies: (1) the polysaccharide-AH derivative will continuously react with the polysaccharide activated by cyanogen bromide to form a self-polymer of the polysaccharide, so that the conjugation efficiency of the polysaccharide to the protein is reduced; (2) EDAC is easy to cause self-crosslinking of the carrier protein while mediating the conjugation of the ADH-derived polysaccharide to the carrier protein, thereby reducing the conjugation efficiency of the polysaccharide to the protein. Therefore, the immunogenicity and antibody persistence of the polysaccharide-protein conjugate vaccine still need to be further improved; for example, vaccination with polysaccharide conjugate vaccine needs to be performed three times to generate immune effect. These deficiencies have limited further development of polysaccharide conjugate vaccines.

Modification in biomolecular systems using the Click reaction of azido and alkynyl is a hotspot of research in recent years. Natalie W. Nairn et al. introduced an unnatural amino acid containing azido into interferon β using a methionine-deficient strain, followed by single-site PEG modification using a copper-catalyzed Click reaction (Natalie W. Narin et al., Bioconjugate Chem. 2012).

In recent years, the genetic code expansion technology develops rapidly. By using an amber stop codon as a sense codon and by introducing corresponding orthogonal tRNA and aminoacyl tRNAsynthetase, a designed unnatural amino acid can be finally introduced into a protein, so that special functions can be endowed to the protein based on the properties of the unnatural amino acid. So far, this technology has successfully and site-directly expressed several dozen kinds of unnatural amino acids in the proteins of living cells, and the involved unnatural amino acids comprise alkynyl, azido, etc. Site-directed modification of proteins can be made specifically by utilizing these special groups that do not originally exist in organisms. In order to solve such problems in the prior art as inhomogeneity in the process for conjugate vaccines and complex separation and purification process, a method capable of specifically modifying functional groups at any site is urgently needed in the field.

CN106929482A describes influenza virus with site-directed mutation, live vaccine thereof, preparation method therefor and use thereof, and the modified carrier protein Hin47-HID of the present invention has been proved to be capable of preventing non-invasive *Haemophilusinfluenzae.* In a specific embodiment of the present invention, provided are a mutant carrier protein Hin47-HID site-directedly introduced with a non-natural amino acid and a method for performing site-directed modification thereof. In this mutation method, the specific azido of the unnatural amino acid Lys-azido introduced at a specific site in the protein can specifically be in Click reaction with a modifier and thereby site-directly couples the non-natural amino acid to the target protein.

### SUMMARY

The present invention relates to an immunogenic composition with site-directed mutation and site-directed modification, a preparation method therefor and use thereof. For the immunogenic composition, an amber codon TAG is introduced at a specific site of a carrier protein gene, and orthogonal aminoacyl tRNAsynthetase-tRNA is used to site-directly mutate an unnatural amino acid with cross-linking property to the specific site of the carrier protein. In a mutual reaction process of the carrier protein and a polysaccharide antigen, a covalent bond is formed, and meanwhile a formed conjugate is in a bead-string state, so that the carrier protein and the polysaccharide antigen can be effectively prevented from being excessively crosslinked. Further, particle size distribution of the conjugate is significantly uniform and controllable, which provides an effective means for improving quality of a polysaccharide-protein conjugate vaccine. Based on thinking and research on the prior art, the inventor utilizes a protein translation system of tRNA (tRNA^{Pyl}) and pyrrolysine-tRNAsynthetase (tRNA^{Pyl}/PylRS) of *Methanococcus archaea* to allow an unnatural amino acid to be site-directly incorporated into a protein, so as to obtain a target peptide or protein with site-directed mutation, such as HID-Hin47, Hin47-HID or CRM197, and then the carrier protein with site-directed mutation, as a raw material which can be further site-directly modified, is conjugated to a polysaccharide to obtain a polysaccharide vaccine of a carrier protein with site-directed modification at a single site.

Accordingly, the present invention provides a carrier protein with site-directed mutation, wherein the carrier protein is selected from fusion proteins formed by one, two or more of diphtheria toxoid, a non-toxic mutant of diphtheria toxin, a bacterial outer membrane protein and a bacterially expressed protein, wherein an amino acid at at least one site on the carrier protein is mutated into an unnatural amino acid, and the unnatural amino acid contains an azido or alkynyl terminal group.

The carrier protein is selected from fusion proteins formed by one, two or more of diphtheria toxoid, non-toxic mutant of diphtheria toxin CRM197 (SEQ ID NO: 1), group B meningococcal outer membrane protein, recombinant *Pseudomonas aeruginosa* exotoxin A, *Haemophilusinfluenzae* lipoprotein HID and *Haemophilusinfluenzae* heat shock protein HIN47. Preferably, the carrier protein is selected from *Haemophilusinfluenzae* lipoprotein HID, fusion protein HIN47-HID and fusion protein HID-HIN47.

The unnatural amino acid is a phenylalanine derivative, a tyrosine derivative, a glutamine derivative, an alanine derivative, a cysteine derivative, a serine derivative or a lysine derivative. Preferably, the unnatural amino acid is a lysine derivative containing azido. More preferably, the unnatural amino acid is:

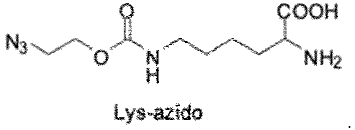

In one embodiment of the present invention, the carrier protein is the HIN47-HID fusion protein, and the mutation site may be one or more amino acids at any site in SEQ ID NO: 2; preferably, the mutation site is selected from position 174R, position 2171, position 223N, position 2261, position 313R, position 315K, position 522K, position 557D, position 653E, position 684Y and other sites which have small influence on activity on a sequence set forth in SEQ ID NO: 2.

In one embodiment of the present invention, the carrier protein is the HID-HIN47 protein, and the mutation site may be one or more amino acids at any site in SEQ ID NO: 3; preferably, the mutation site is selected from position 78K, position 113D, position 209E, position 240Y, position 527R, position 5701, position 576N, position 5791, position 666R, position 668K and other sites which have small influence on activity on a sequence set forth in SEQ ID NO: 3. After mutation, the difference between the amino acid sequence of the protein with site-directed mutation and the amino acid sequence of the target protein before mutation is that the amino acid at position N on the amino acid sequence of the protein before mutation is mutated into Lys-azido, and the linking mode of the mutated amino acid is shown as the following formula: 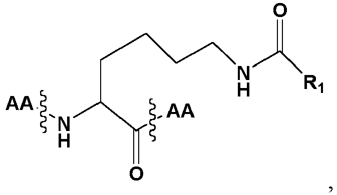 wherein, the N is a mutation site, and AA is an amino acid 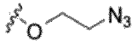 located before and after the mutation site.

The present invention also provides a conjugate of a carrier protein with site-directed mutation, wherein the conjugate is prepared by the carrier protein with site-directed mutation of the present invention and a molecule containing or modified to have an alkynyl terminal group, wherein the molecule is a saccharide, a polynucleotide, an amino acid, a polypeptide or a small molecule compound containing an alkynyl terminal group, or is a modified substance obtained by modification of a saccharide, a polynucleotide, an amino acid, a polypeptide or a small molecule compound with an alkynyl terminal group.

The carrier protein with site-directed mutation of the present invention and a molecule containing or modified to have an alkynyl terminal group are subjected to a Click reaction to obtain the conjugate. The Click reaction can be a Click reaction mediated by monovalent copper and can also be a copper-free Click reaction mediated by cyclooctyne or a derivative thereof. The saccharide, the polynucleotide, the amino acid, the polypeptide or the small molecular compound of the present invention can be a modified substance obtained by modification of a saccharide, a polynucleotide, an amino acid, a polypeptide or a small molecule compound with an alkynyl terminal group, which realizes site-directed coupling by the catalysis of monovalent copper catalyst to obtain the conjugate. Alternatively, the saccharide, the polynucleotide, the amino acid, the polypeptide or the small molecular compound is a modified substance with cyclooctyne or a derivative thereof as a modifying group, which directly realizes site-directed coupling.

Preferably, in the conjugate of the carrier protein with site-directed mutation of the present invention, the amino acid at position N on the amino acid sequence of the protein before mutation is mutated into the following structure: 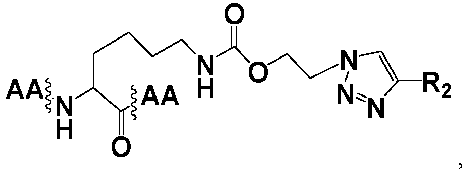 or 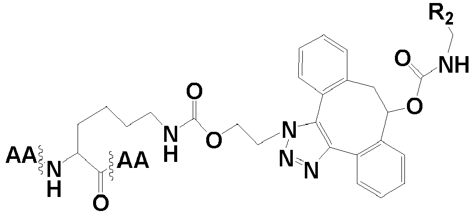 wherein,
the N is a mutation site, AA is an amino acid located before and after the mutation site, and
R₂ is a saccharide, a polynucleotide, an amino acid, a polypeptide or a carboxyl terminal modifying group.

In one embodiment of the present invention, the conjugate is a glycoconjugate formed by conjugating the carrier protein with site-directed mutation of the present invention to a polysaccharide containing or modified to have an alkynyl terminal group.

Preferably, the protein with site-directed mutation of the present invention is conjugated to a polysaccharide, wherein the ratio of the polysaccharide to the carrier protein (w/w) is 0.3 to 3.

Preferably, at least one covalent bond is present between the polysaccharide and the carrier protein every 10 to 50 saccharide repeat units of the polysaccharide.

The polysaccharide of the present invention can be a modified substance obtained by modification with an alkynyl terminal group, which realizes site-directed coupling by the catalysis of monovalent copper catalyst to obtain a glycoconjugate. Alternatively, the polysaccharide is a modified substance with cyclooctyne or a derivative thereof as a modifying group, which directly realizes site-directed coupling to a target polysaccharide protein.

The present application also provides an immunogenic composition comprising the glycoconjugate of the present invention and a pharmaceutically acceptable excipient, carrier or diluent.

The polysaccharide is selected from capsular polysaccharide; for example, the capsular polysaccharide is one of or a combination of two or more of pneumococcus serotype 1, 2, 3, 4, 5, 6A, 6B, 7F, 8, 9N, 9V, 10A, 11A, 12F, 14, 15B, 17F, 18C, 19A, 19F, 20, 22F, 23F and 33F.

The present invention also provides a multivalent conjugate vaccine for pneumonia formed by multivalent pneumococcal polysaccharide and two or more carrier proteins with site-directed mutation of the present invention.

The present invention also provides use of the carrier protein with site-directed mutation, the conjugate of the carrier protein with site-directed mutation or the immunogenic composition in preparation of a vaccine.

Preferably, the vaccine is an influenza vaccine or a pneumonia vaccine.

Compared with other methods, the advantages of the present invention may be embodied in one or more of the following:
1. An unnatural amino acid can be introduced at any site of a protein, thereby creating a carrier protein that can be specifically modified at this site only;
2. Efficient and specific modification can be realized by utilizing the specific active group on the unnatural amino acid;
3. The modification of a specific site can weaken the activity of protease with specific functions, and thus reduce side effects and realize the effect of the carrier protein;
4. By optimizing modification conditions and utilizing cyclooctyne-mediated copper-free Click reaction, a modification reaction that is high in efficiency, harmless to proteins and simple and feasible can be realized.

### BRIEF DESCRIPTION OF THE DRAWINGS

FIG. 1 shows SDS-PAGE electrophoretograms of the mutated protein Hin47-HID and the polysaccharide-protein conjugates obtained by Click reaction;
FIG. 2a shows a particle size distribution plot of the polysaccharide-protein conjugate obtained by Click reaction;
FIG. 2b shows a particle size distribution plot of the polysaccharide-protein conjugate obtained by conventional coupling method; and
FIG. 3 shows ELISA results of type 3 conjugates obtained by different coupling modes after triple immune of rabbits.

### DETAILED DESCRIPTION

The present invention will be further illustrated with reference to the following specific examples. It should be understood that these examples are merely intended to illustrate the present invention rather than limit the scope of the present invention.

### Example 1 Construction of Expression Plasmid of Carrier Protein with Site-directed Mutation

### 1: Selection of mutation sites

The three-dimensional structures of HID and HIN47 were predicted separately using online software phyre2.

10 key amino acids were selected on each of fusion proteins HIN47-HID and HID-HIN47. After the key amino acid sites were substituted, the fusion proteins can be bound to the polysaccharide at fixed points, and the residual protease activity of HIN47 is expected to be further reduced as well, which is beneficial to improve the stability of a final polysaccharide-binding product. The information of specific mutation sites is shown in Table 1 and Table 2, wherein an amino acid position refers to a position on sequences set forth in SEQ ID NOs: 2-3.

**Table 1. HIN47-HID mutation sites**

| Amino acid position | Amino acid | Codon before mutation | Codon after mutation |
|---|---|---|---|
| 522 | K | AAG | TAG |
| 557 | D | GAC | |
| 653 | E | GAA | |
| 684 | Y | TAT | |
| 174 | R | CGC | |
| 217 | I | ATC | |
| 223 | N | AAT | |
| 226 | I | ATT | |
| 313 | R | CGC | |
| 315 | K | AAA | |

**Table 2. HID-HIN47 mutation sites**

| Amino acid position | Amino acid | Codon before mutation | Codon after mutation |
|---|---|---|---|
| 78 | K | AAG | TAG |
| 113 | D | GAC | |
| 209 | E | GAA | |
| 240 | Y | TAT | |
| 527 | R | CGC | |
| 570 | I | ATC | |
| 576 | N | AAT | |
| 579 | I | ATT | |
| 666 | R | CGC | |
| 668 | K | AAA | |

### 2: Obtaining of expression plasmids

According to HID and HIN47 gene sequences published by NCBI GeneBank, full-length DNA fragments of the genes were each obtained through whole-gene synthesis. Then HIN47 and HID proteins were each fused and constructed, by using a GGSGGS six-amino-acid linker, in a pET9a vector in sequences of HIN47-linker-HID and HID-linker-HIN47, respectively, so that expression plasmids pET9a-HIN47-HID and pET9a-HID-HIN47 were obtained, respectively.

### 3: Site-directed mutation

The Fast Mutagenesis System site-directed mutation kit from TransGene Biotech was used, and according to the instruction of the kit, the expression plasmids pET9a-HIN47-HID and pET9a-HID-HIN47 were used as templates, and the mutation primers in Tables 3-4 were used for mutations. For the plasmids obtained after the mutation, sequencing verification was performed. Sequencing results showed that all genes at mutation sites were successfully mutated into TAG, and 10 plasmids with site-directed mutation were obtained.

The 10 mutant clones of the fusion protein pET9a-HIN47-HID were named: pET9a-HIN47-HID-R174, pET9a-HIN47-HID-I217, pET9a-HIN47-HID-N223, pET9a-HIN47-HID-I226, pET9a-HIN47-HID-R313, pET9a-HIN47-HID-K315, pET9a-HIN47-HID-K522, pET9a-HIN47-HID-D557, pET9a-HIN47-HID-E653, and pET9a-HIN47-HID-Y684.

The 10 mutant clones of the fusion protein pET9a-HID-HIN47 were named:
pET9a-HID-HIN47-K78, pET9a-HID-HIN47-D113, pET9a-HID-HIN47-E209, pET9a-HID-HIN47-Y240, pET9a-HID-HIN47-R527, pET9a-HID-HIN47-I570, pET9a-HID-HIN47-N576, pET9a-HID-HIN47-I579, pET9a-HID-HIN47-R666, and pET9a-HID-HIN47-K668.

**Table 3. List of mutation primers for HIN47-HID**

| Mutation site | Primer coding | Sequence number | Primer sequence 5' to 3' |
|---|---|---|---|
| K522 | 522F | SEQ ID NO: 4 | CCGATGTAGCCtagAAATTTCCTCATAGGCATAGGAAAG |
| | 522R | SEQ ID NO: 5 | TTTctaGGCTACATCGGTTAAGCCGTCCAGAA |
| D557 | 557F | SEQ ID NO: 6 | GACCAAAtagGGGAAACAGGCTCAAGTGTATCC |
| | 557R | SEQ ID NO: 7 | GTTTCCCctaTTTGGTCTCGAAATTTTCCGTC |
| E653 | 653F | SEQ ID NO: 8 | AAAACCtagCTCCTTCCTCAGATGGGAATGGA |
| | 653R | SEQ ID NO: 9 | GGAAGGAGctaGGTTTTTATGCGTTTCAGTTCATT |
| Y684 | 684F | SEQ ID NO: 10 | CGAAAGGAtagTGGGTAAACTACAATTATGATTGGATG |
| | 684R | SEQ ID NO: 11 | TACCCActaTCCTTTCGGATCTTTTTCCTGGG |
| R174 | 174F | SEQ ID NO: 12 | tagAGTACCGGGTCAGACTCCGGCACCTACGA |
| | 174R | SEQ ID NO: 13 | TCTGACCCGGTACTctaGCCCAGAGCGGAAACTATCC |
| 1217 | 217F | SEQ ID NO: 14 | TGCTATCtagTCACCGAGCGGCGGTAATGCGG |
| | 217R | SEQ ID NO: 15 | TCGGTGActaGATAGCAGTATTGATACCGATCAATTC |
| N223 | 223F | SEQ ID NO: 16 | TtagGCGGGAATTGCGTTCGCAATTCCCAGTA |
| | 223R | SEQ ID NO: 17 | ACGCAATTCCCGCctaACCGCCGCTCGGTGAGAT |
| 1226 | 226F | SEQ ID NO: 18 | AATGCGGGAtagGCGTTCGCAATTCCCAGTAA |
| | 226R | SEQ ID NO: 19 | AACGCctaTCCCGCATTACCGCCGCTCGGTGA |
| R313 | 313F | SEQ ID NO: 20 | GCTGAAATTtagGCAAAAATTGCTACAACTGGTGC |
| | 313R | SEQ ID NO: 21 | TTTGCctaAATTTCAGCAAACGAAGAGATTTTC |
| K315 | 315F | SEQ ID NO: 22 | TCGCGCAtagATTGCTACAACTGGTGCAGGCA |
| | 315R | SEQ ID NO: 23 | TAGCAATctaTGCGCGAATTTCAGCAAACGAA |

**Table 4. List of mutation primers for HID-HIN47**

| Mutation site | Primer coding | Sequence number | Primer sequence 5' to 3' |
|---|---|---|---|
| K78 | 78F | SEQ ID NO: 24 | CCGATGTAGCCtagAAATTTCCTCATAGGCATAGGAAAG |
| | 78R | SEQ ID NO: 25 | TTTctaGGCTACATCGGTTAAGCCGTCCAGAA |
| D113 | 113F | SEQ ID NO: 26 | GACCAAAtagGGGAAACAGGCTCAAGTGTATCC |
| | 113R | SEQ ID NO: 27 | GTTTCCCctaTTTGGTCTCGAAATTTTCCGTC |
| E209 | 209F | SEQ ID NO: 28 | AAAACCtagCTCCTTCCTCAGATGGGAATGGA |
| | 209R | SEQ ID NO: 29 | GGAAGGAGctaGGTTTTTATGCGTTTCAGTTCATT |
| Y240 | 240F | SEQ ID NO: 30 | CGAAAGGAtagTGGGTAAACTACAATTATGATTGGATG |
| | 240R | SEQ ID NO: 31 | TACCCActaTCCTTTCGGATCTTTTTCCTGGG |
| R527 | 527F | SEQ ID NO: 32 | tagAGTACCGGGTCAGACTCCGGCACCTACGA |
| | 527R | SEQ ID NO: 33 | TCTGACCCGGTACTctaGCCCAGAGCGGAAACTATCC |
| 1570 | 570F | SEQ ID NO: 34 | TGCTATCtagTCACCGAGCGGCGGTAATGCGG |
| | 570R | SEQ ID NO: 35 | TCGGTGActaGATAGCAGTATTGATACCGATCAATTC |
| N576 | 576F | SEQ ID NO: 36 | TtagGCGGGAATTGCGTTCGCAATTCCCAGTA |
| | 576R | SEQ ID NO: 37 | ACGCAATTCCCGCctaACCGCCGCTCGGTGAGAT |
| 1579 | 579F | SEQ ID NO: 38 | AATGCGGGAtagGCGTTCGCAATTCCCAGTAA |
| | 579R | SEQ ID NO: 39 | AACGCctaTCCCGCATTACCGCCGCTCGGTGA |
| R666 | 666F | SEQ ID NO: 40 | GCTGAAATTtagGCAAAAATTGCTACAACTGGTGC |
| | 666R | SEQ ID NO: 41 | TTTGCctaAATTTCAGCAAACGAAGAGATTTTC |
| K668 | 668F | SEQ ID NO: 42 | TCGCGCAtagATTGCTACAACTGGTGCAGGCA |
| | 668R | SEQ ID NO: 43 | TAGCAATctaTGCGCGAATTTCAGCAAACGAA |

### Example 2 Expression and Purification of Mutant Carrier Protein

### Lys-azido incorporation expression and purification of mutant proteins

The expression plasmid pET9a-HIN47-HID-K522 obtained in Example 1 was cultured in LB medium at 37 °C for 12 to 16 h, and then secondary amplification was carried out until the OD value of the bacterial solution was 0.6 to 1.0. Lys-azido was added until a final concentration of 1 mM was reached, and amplification was continued at 37 °C for 30 min, and IPTG was added until a final concentration of 0.5 mM was reached. The final concentration of arabinose was 0.2%. Expression was induced at 24 °C for 12 h, and then bacteria were collected.

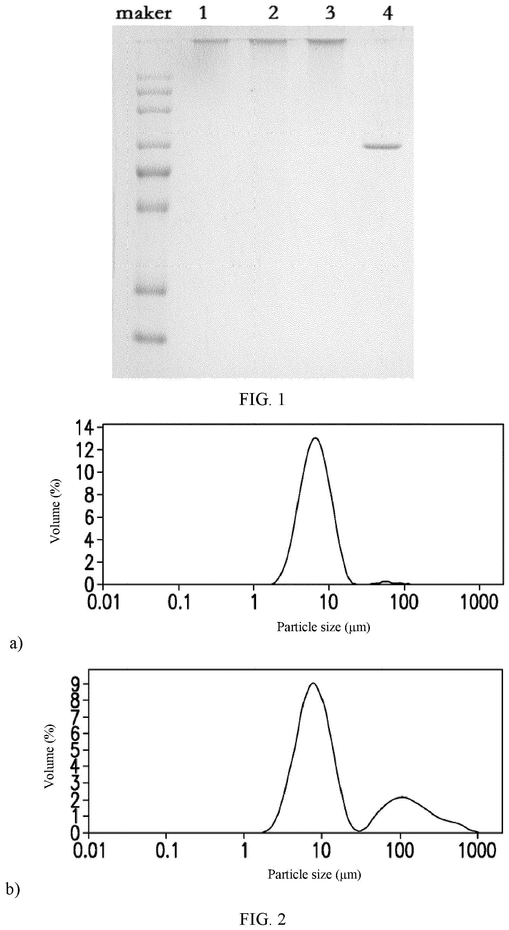

The collected bacteria were equilibrated and resuspended using Ni-NTA-Bind-Buffer, disrupted by ultrasonic, centrifuged to remove cell debris, purified by Ni-NTA metal chelating affinity chromatography, fully washed with Ni-NTA-Wash-Buffer and finally eluted with Ni-NTA-Elute-Buffer to obtain a preliminary purified protein sample HIN47-HID-K522 with a purity of about 90% (see FIG. 1, line 4).

### Example 3 Site-Directed Coupling of Mutant Carrier Protein to Polysaccharide by Copper-Free Catalysis

The copper-free Click reaction needed to rely on the ring tension effect of cyclooctyne, and a modified substance was coupled to DIBO (polysaccharide with a cyclooctyne structure), so that the copper-free catalytic Click reaction with a group containing azido was carried out.

The Click reaction system was as follows: 1 µg/µL HIN47-HID-K522 protein and DIBO-pneumonia type 3 polysaccharide (a molecular weight of 200 KD and 400 KD, 2 mM each) were vertically suspended at 4 °C for 2 hours. The results showed that pneumonia type 3 polysaccharide with different molecular weights (200 KD and 400 KD) could be successfully conjugated to the protein by modification to obtain F3-HIN47-HID-K522-200 and F3-HIN47-HID-K522-400 (see FIG. 1, lines 1 and 2).

By the reaction conditions described above, about 90% of proteins could be site-directly coupled with polysaccharides within 1 h. The re-dissolved substance after the reaction was desalted and then subjected to ion exchange, so that a protein with site-directedly coupled polysaccharide that had a purity > 95% could be obtained.

### Example 4Coupling to Polysaccharide by Copper-Catalyzed Click Reaction

The reaction system was as follows:

| | |
|---|---|
| HIN47-HID-K522 protein | 1 µg/µL |
| Pneumonia type 1 polysaccharide-alkyne | 20 µg/µL |
| Cu²⁺ | 1 mM |
| BTTES | 400µmol |
| PBS | 0.01 M (pH ≈ 7) |

Cu wire segment (sufficient amount)

Note: (BTTES was short for (1,2,3-triazol-1-yl) ethanesulfonic acid)

The reaction conditions were as follows: the reaction system was vertically suspended for 30 min at 4 °C, and after the reaction was finished, EDTA was added until a concentration of 1 mM was reached to stop the reaction. The resulting final product was a conjugate F3-HIN47-HID-K522-Cu that was formed by site-directly coupling the pneumonia type 3 polysaccharide to the HIN47-HID-K522 protein (see FIG. 1, line 3), wherein the coupling of polysaccharide to the target protein is carried out through a copper-catalyzed Click reaction, as can be seen in FIG. 1.

### Example 5 Evaluation of Filterability of Protein with Site-Directedly Coupled Polysaccharide

According to analysis of the particle size distribution of the conjugates using the Malvern laser particle size analyzer Mastersizer 2000, the particle size distribution of the conjugate formulation obtained by site-directly coupling polysaccharide to protein was uniform and free of obvious aggregates, but the conjugate F3-HIN47-HID-K522-Cu obtained by the conventional polysaccharide-protein coupling method was not uniform and contained aggregates (see FIG. 2b). The conventional polysaccharide-protein coupling method was as follows: type 3 polysaccharide was hydrolyzed at high temperature of 60 °C and then subjected to activation reaction in the presence of 0.5 g/L sodium periodate to obtain an activated polysaccharide; the activated polysaccharide mixed with carrier protein HIN47-HID in 0.1 M phosphate buffer solution (pH 6.5), sodium cyanoborohydride was added until a final concentration of 2 g/L was reached, and the reaction mixture reacted for 56 h; then 2 g/L sodium borohydride, was added, and the reaction was continued for two hours; and then ultrafiltration with normal saline was performed to obtain a conjugate F3-HIN47-HID.

### Example 6 Evaluation on Immunogenicity of Protein Conjugate with Site-Directedly Coupled Polysaccharide

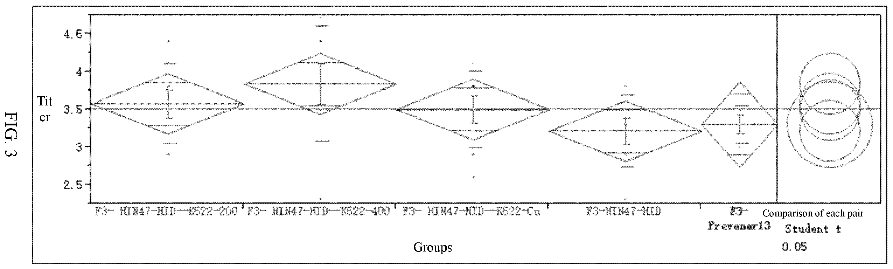

In the rabbit models, vaccines obtained by different coupling modes were studied. The study related to the effects of the polysaccharide-protein conjugates of the type 3 capsular polysaccharide from *Streptococcus pneumoniae* and the HIN47-HID proteins in Examples 3, 4 and 5 and aluminium phosphate (AlPO₄) adjuvant on the immune response of a monovalent vaccine in rabbits. These effects were characterized by serum IgG concentrations determined by antigen-specific ELISA and antibody functions determined by opsonophagocytic assay (OPA). FIG. 3 shows ELISA results of type 3 conjugates obtained by different coupling modes after triple immune of rabbits. The results show that polysaccharide-protein conjugates of the type 3 capsular polysaccharide from *Streptococcus pneumoniae* and HIN47-HID proteins in Examples 3, 4, and 5 showed significant antibody titers compared to the control.

At weeks 0, 2, and 4, New Zealand white rabbits were immunized intramuscularly with one-quarter dose (0.5 µg of polysaccharide) of the planned clinical dose (2 µg of polysaccharide) for human. Serums were collected at different time points. Serum-specific IgG was determined by ELISA and functional activity was assessed by bactericidal ability (OPA).

**Table 5. Detection results of type 3 polysaccharide-protein conjugates**

| Polysaccharide-protein conjugate | Polysaccharide-protein conjugation ratio | Free polysaccharide (%) | Free protein (%) |
|---|---|---|---|
| F3-HIN47-HID--K522-200 | 0.62 | 18 | 1.2 |
| F3-HIN47-HID--K522-400 | 0.98 | 12 | 1 |
| F3-HIN47-HID--K522-Cu | 0.85 | 16 | 1.6 |
| F3-HIN47-HID | 0.89 | 23 | 2.8 |

**Table 6. Bactericidal ability (OPA) results of type 3 polysaccharide-protein conjugates**

| Polysaccharide-protein conjugate | OPA50%) |
|---|---|
| F3- HIN47-HID--K522-200 | 698 |
| F3- HIN47-HID--K522-400 | 1098 |
| F3- HIN47-HID--K522-Cu | 773 |
| F3-HIN47-HID | 305 |
| F3-prevenar | 325 |

Table 5 shows the detection results of the type 3 polysaccharide-protein conjugates, and the results show that the type 3 polysaccharide-protein conjugates obtained by site-directed coupling have percentages of free polysaccharide and free protein that are significantly less than those of the unmutated protein carrier. Table 6 shows the bactericidal ability (OPA) results of the type 3 polysaccharide-protein conjugates, and the results show that the type 3 polysaccharide-protein conjugates obtained by site-directed coupling induce significantly higher antibody responses and specific bactericidal ability in rabbits.

### Example 7 Study on Safety of Protein Conjugate with Site-Directedly Coupled Polysaccharide

3 polysaccharide-protein conjugates prepared in Examples 3 and 4 were sampled. 5 mice weighed 18-22 g and 2 guinea pigs weighed 250-350 g were selected for each sample, wherein each mouse was subcutaneously injected with 0.5 mL of sample at ventral area and each guinea pig was subcutaneously injected with 5.0 mL of sample at ventral area. The animals were observed for 7 days, and the body weight changes thereof were recorded. The results show that all the mice and the guinea pigs were healthy and had no abnormal reaction in the observation period, and the body weight of each mouse and guinea pig was increased at the end of observation period compared to that before injection. This shows that the site-directed coupling conjugate vaccines prepared by the present invention haven't been contaminated by exogenous toxic substances and they are free of unexpected unsafe factors.

Examples 2-7 take HIN47-HID-K522 as an example to demonstrate effect experiments of preparation, characterization, detection methods, immunogenicity evaluation and the like of the mutant carrier protein. Other carrier proteins with site-directed mutation of HIN47-linker-HID and HID-linker-HIN47 are prepared by the methods above, and after immunogenicity and safety evaluation, they can reach a level equivalent to HIN47-HID-K522. The results show that the type 3 polysaccharide-protein conjugates obtained by site-directed coupling induce significantly higher antibody responses and specific bactericidal ability in rabbits.

Although the present invention has been described with the preferred embodiments above, they are not intended to limit the scope of the claims. Since many possible variations and modifications can be made by one skilled in the art without departing from the conception of the present invention, the scope of the present invention is to be determined by the claims.

## Claims

1. A carrier protein with site-directed mutation, wherein the carrier protein is selected from fusion proteins formed by one, two or more of diphtheria toxoid, a non-toxic mutant of diphtheria toxin, a bacterial outer membrane protein and a bacterially expressed protein, wherein an amino acid at at least one site on the carrier protein is mutated into an unnatural amino acid, and the unnatural amino acid contains an azido or alkynyl terminal group.

2. The protein with site-directed mutation of claim 1, wherein the carrier protein is selected from fusion proteins formed by one, two or more of diphtheria toxoid, non-toxic mutant of diphtheria toxin CRM197, group B meningococcal outer membrane protein, recombinant *Pseudomonas aeruginosa* exotoxin A, *Haemophilusinfluenzae* lipoprotein HID and*Haemophilusinfluenzae* heat shock protein HIN47; preferably, the carrier protein is selected from non-toxic mutant CRM197 of diphtheria toxin, *Haemophilusinfluenzae* lipoprotein HID, fusion protein HIN47-HID and fusion protein HID-HIN47.

3. The protein with site-directed mutation of claim 1, wherein the unnatural amino acid is a phenylalanine derivative, a tyrosine derivative, a glutamine derivative, an alanine derivative, a cysteine derivative, a serine derivative or a lysine derivative.

4. The protein with site-directed mutation of claim 1, wherein the unnatural amino acid is

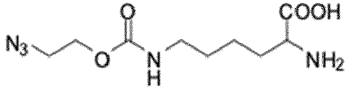

5. The protein with site-directed mutation of claim 1, wherein a mutation site of HIN47-HID fusion protein may be one or more amino acids at any site in SEQ ID NO: 2; preferably, the mutation site is selected from position 174R, position 2171, position 223N, position 2261, position 313R, position 315K, position 522K, position 557D, position 653E and position 684Y on a sequence set forth in SEQ ID NO: 2.

6. The protein with site-directed mutation of claim 1, wherein a mutation site of HID-HIN47 fusion protein may be one or more amino acids at any site in SEQ ID NO: 3; preferably, the mutation site is selected from position 78K, position 113D, position 209E, position 240Y, position 527R, position 5701, position 576N, position 5791, position 666R and position 668K on a sequence set forth in SEQ ID NO: 3.

7. The protein with site-directed mutation of any one of claims 1-7, wherein an amino acid at position N on an amino acid sequence of a protein before mutation is mutated into Lys-azido, and a linking mode of the mutated amino acid is shown as the following formula: 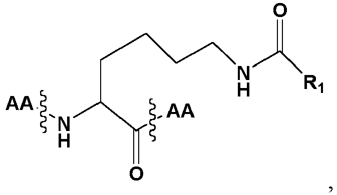 wherein, the N is a mutation site, and AA is an amino acid located before and after the mutation site.

8. A conjugate of a carrier protein with site-directed mutation, wherein the conjugate is prepared by the carrier protein with site-directed mutation of any one of claims 1-8, and a saccharide, a polynucleotide, an amino acid, a polypeptide or a small molecule compound containing or modified to have an alkynyl terminal group.

9. The conjugate of the carrier protein with site-directed mutation of claim 8, wherein the amino acid at position N on the amino acid sequence of the protein before mutation is mutated into the following structure: 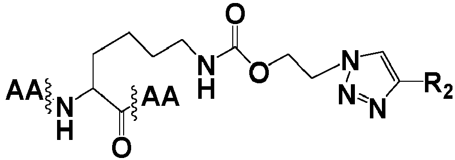 or, 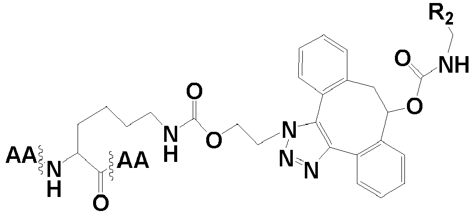 wherein,
the N is a mutation site, AA is an amino acid located before and after the mutation site, and
R₂ is a saccharide, a polynucleotide, an amino acid, a polypeptide or a carboxyl terminal modifying group.

10. The conjugate of the carrier protein with site-directed mutation of claim 8 or 9, wherein the conjugate is a glycoconjugate formed by conjugating the carrier protein with site-directed mutation of any one of claims 1-8 to a polysaccharide containing or modified to have an alkynyl terminal group.

11. The conjugate of the carrier protein with site-directed mutation of claim 10, wherein the ratio of the saccharide to the carrier protein (w/w) in the glycoconjugate is 0.3 to 3.

12. The conjugate of the carrier protein with site-directed mutation of claim 10 or 11, wherein at least one covalent bond is present between the polysaccharide and the carrier protein every 10 to 50 saccharide repeat units of the polysaccharide.

13. An immunogenic composition comprising the glycoconjugate of any one of claims 11-12 and a pharmaceutically acceptable excipient, carrier or diluent.

14. The immunogenic composition of claim 13, wherein the glycoconjugate is a glycoconjugate of a capsular polysaccharide selected from one of or a combination of two or more of: pneumococcus serotype 1, 2, 3, 4, 5, 6A, 6B, 7F, 8, 9N, 9V, 10A, 11A, 12F, 14, 15B, 17F, 18C, 19A, 19F, 20, 22F, 23F and 33F.

15. A multivalent conjugate vaccine for pneumonia formed by multivalent pneumococcal polysaccharide and two or more carrier proteins with site-directed mutation of any one of claims 1-8.

16. Use of the carrier protein with site-directed mutation of any one of claims 1-8, the conjugate of the carrier protein with site-directed mutation of any one of claims 9-13 or the immunogenic composition of any one of claims 14-15 in preparation of a vaccine.

17. The use of claim 16, wherein the vaccine is an influenza vaccine or a pneumonia vaccine.


SEQUENCE LISTING

<110>  CANSINO BIOLOGICS INC.

<120>  CARRIER PROTEIN WITH SITE-DIRECTED MUTATION AND USE THEREOF IN
       PREPARATION OF VACCINE

<150>  CN201910978406.X
<151>  2019-10-15

<160>  43

<170>  PatentIn version 3.5

<210>  1
<211>  560
<212>  PRT
<213>  Artificial Sequence

<220>
<223>  CRM197

<400>  1

Met Ser Arg Lys Leu Phe Ala Ser Ile Leu Ile Gly Ala Leu Leu Gly
1               5                   10                  15

Ile Gly Ala Pro Pro Ser Ala His Ala Gly Ala Asp Asp Val Val Asp
            20                  25                  30

Ser Ser Lys Ser Phe Val Met Glu Asn Phe Ser Ser Tyr His Gly Thr
        35                  40                  45

Lys Pro Gly Tyr Val Asp Ser Ile Gln Lys Gly Ile Gln Lys Pro Lys
    50                  55                  60

Ser Gly Thr Gln Gly Asn Tyr Asp Asp Asp Trp Lys Glu Phe Tyr Ser
65                  70                  75                  80

Thr Asp Asn Lys Tyr Asp Ala Ala Gly Tyr Ser Val Asp Asn Glu Asn
                85                  90                  95

Pro Leu Ser Gly Lys Ala Gly Gly Val Val Lys Val Thr Tyr Pro Gly
            100                 105                 110

Leu Thr Lys Val Leu Ala Leu Lys Val Asp Asn Ala Glu Thr Ile Lys
        115                 120                 125

Lys Glu Leu Gly Leu Ser Leu Thr Glu Pro Leu Met Glu Gln Val Gly
    130                 135                 140

Thr Glu Glu Phe Ile Lys Arg Phe Gly Asp Gly Ala Ser Arg Val Val
145                 150                 155                 160

Leu Ser Leu Pro Phe Ala Glu Gly Ser Ser Ser Val Glu Tyr Ile Asn
                165                 170                 175

Asn Trp Glu Gln Ala Lys Ala Leu Ser Val Glu Leu Glu Ile Asn Phe
            180                 185                 190

Glu Thr Arg Gly Lys Arg Gly Gln Asp Ala Met Tyr Glu Tyr Met Ala
        195                 200                 205

Gln Ala Cys Ala Gly Asn Arg Val Arg Arg Ser Val Gly Ser Ser Leu
    210                 215                 220

Ser Cys Ile Asn Leu Asp Trp Asp Val Ile Arg Asp Lys Thr Lys Thr
225                 230                 235                 240

Lys Ile Glu Ser Leu Lys Glu His Gly Pro Ile Lys Asn Lys Met Ser
                245                 250                 255

Glu Ser Pro Asn Lys Thr Val Ser Glu Glu Lys Ala Lys Gln Tyr Leu
            260                 265                 270

Glu Glu Phe His Gln Thr Ala Leu Glu His Pro Glu Leu Ser Glu Leu
        275                 280                 285

Lys Thr Val Thr Gly Thr Asn Pro Val Phe Ala Gly Ala Asn Tyr Ala
    290                 295                 300

Ala Trp Ala Val Asn Val Ala Gln Val Ile Asp Ser Glu Thr Ala Asp
305                 310                 315                 320

Asn Leu Glu Lys Thr Thr Ala Ala Leu Ser Ile Leu Pro Gly Ile Gly
                325                 330                 335

Ser Val Met Gly Ile Ala Asp Gly Ala Val His His Asn Thr Glu Glu
            340                 345                 350

Ile Val Ala Gln Ser Ile Ala Leu Ser Ser Leu Met Val Ala Gln Ala
        355                 360                 365

Ile Pro Leu Val Gly Glu Leu Val Asp Ile Gly Phe Ala Ala Tyr Asn
    370                 375                 380

Phe Val Glu Ser Ile Ile Asn Leu Phe Gln Val Val His Asn Ser Tyr
385                 390                 395                 400

Asn Arg Pro Ala Tyr Ser Pro Gly His Lys Thr Gln Pro Phe Leu His
                405                 410                 415

Asp Gly Tyr Ala Val Ser Trp Asn Thr Val Glu Asp Ser Ile Ile Arg
            420                 425                 430

Thr Gly Phe Gln Gly Glu Ser Gly His Asp Ile Lys Ile Thr Ala Glu
        435                 440                 445

Asn Thr Pro Leu Pro Ile Ala Gly Val Leu Leu Pro Thr Ile Pro Gly
    450                 455                 460

Lys Leu Asp Val Asn Lys Ser Lys Thr His Ile Ser Val Asn Gly Arg
465                 470                 475                 480

Lys Ile Arg Met Arg Cys Arg Ala Ile Asp Gly Asp Val Thr Phe Cys
                485                 490                 495

Arg Pro Lys Ser Pro Val Tyr Val Gly Asn Gly Val His Ala Asn Leu
            500                 505                 510

His Val Ala Phe His Arg Ser Ser Ser Glu Lys Ile His Ser Asn Glu
        515                 520                 525

Ile Ser Ser Asp Ser Ile Gly Val Leu Gly Tyr Gln Lys Thr Val Asp
    530                 535                 540

His Thr Lys Val Asn Ser Lys Leu Ser Leu Phe Phe Glu Ile Lys Ser
545                 550                 555                 560

<210> 2
<211> 792
<212> PRT
<213> Artificial Sequence

<220>
<223> HIN47-HID

<400> 2

Met Thr Leu Pro Ser Phe Val Ser Glu Gln Asn Ser Leu Ala Pro Met
1               5                   10                  15

Leu Glu Lys Val Gln Pro Ala Val Val Thr Leu Ser Val Glu Gly Lys
            20                  25                  30

Ala Lys Val Asp Ser Arg Ser Pro Phe Leu Asp Asp Ile Pro Glu Glu
        35                  40                  45

Phe Lys Phe Phe Phe Gly Asp Arg Phe Ala Glu Gln Phe Gly Gly Arg
    50                  55                  60

Gly Glu Ser Lys Arg Asn Phe Arg Gly Leu Gly Ser Gly Val Ile Ile
65                  70                  75                  80

Asn Ala Ser Lys Gly Tyr Val Leu Thr Asn Asn Ala Val Ile Asp Glu
                85                  90                  95

Ala Asp Lys Ile Thr Val Gln Leu Gln Asp Gly Arg Glu Phe Lys Ala
            100                 105                 110

Lys Leu Val Gly Lys Asp Glu Leu Ser Asp Ile Ala Leu Val Gln Leu
        115                 120                 125

Glu Lys Pro Ser Asn Leu Thr Glu Ile Lys Phe Ala Asp Ser Asp Lys
    130                 135                 140

Leu Arg Val Gly Asp Phe Thr Val Ala Ile Gly Asn Pro Phe Gly Leu
145                 150                 155                 160

Gly Gln Thr Val Thr Ser Gly Ile Val Ser Ala Leu Gly Arg Ser Thr
                165                 170                 175

Gly Ser Asp Ser Gly Thr Tyr Glu Asn Tyr Ile Gln Thr Asp Ala Ala
            180                 185                 190

Val Asn Arg Gly Asn Ser Gly Gly Ala Leu Val Asn Leu Asn Gly Glu
        195                 200                 205

Leu Ile Gly Ile Asn Thr Ala Ile Ile Ser Pro Ser Gly Gly Asn Ala
    210                 215                 220

Gly Ile Ala Phe Ala Ile Pro Ser Asn Gln Ala Ser Asn Leu Val Gln
225                 230                 235                 240

Gln Ile Leu Glu Phe Gly Gln Val Arg Arg Gly Leu Leu Gly Ile Lys
                245                 250                 255

Gly Gly Glu Leu Asn Ala Asp Leu Ala Lys Ala Phe Asn Val Ser Ala
            260                 265                 270

Gln Gln Gly Ala Phe Val Ser Glu Val Leu Pro Lys Ser Ala Ala Glu
        275                 280                 285

Lys Ala Gly Leu Lys Ala Gly Asp Ile Ile Thr Ala Met Asn Gly Gln
    290                 295                 300

Lys Ile Ser Ser Phe Ala Glu Ile Arg Ala Lys Ile Ala Thr Thr Gly
305                 310                 315                 320

Ala Gly Lys Glu Ile Ser Leu Thr Tyr Leu Arg Asp Gly Lys Ser His
                325                 330                 335

Asp Val Lys Met Lys Leu Gln Ala Asp Asp Ser Ser Gln Leu Ser Ser
            340                 345                 350

Lys Thr Glu Leu Pro Ala Leu Asp Gly Ala Thr Leu Lys Asp Tyr Asp
        355                 360                 365

Ala Lys Gly Val Lys Gly Ile Glu Ile Thr Lys Ile Gln Pro Asn Ser
    370                 375                 380

Leu Ala Ala Gln Arg Gly Leu Lys Ser Gly Asp Ile Ile Ile Gly Ile
385                 390                 395                 400

Asn Arg Gln Met Ile Glu Asn Ile Arg Glu Leu Asn Lys Val Leu Glu
                405                 410                 415

Thr Glu Pro Ser Ala Val Ala Leu Asn Ile Leu Arg Gly Asp Ser Asn
            420                 425                 430

Phe Tyr Leu Leu Val Gln Gly Gly Ser Gly Gly Ser Met Asp Pro Ser
        435                 440                 445

Ser His Ser Ser Asn Met Ala Asn Thr Gln Met Lys Ser Asp Lys Ile
    450                 455                 460

Ile Ile Ala His Arg Gly Ala Ser Gly Tyr Leu Pro Glu His Thr Leu
465                 470                 475                 480

Glu Ser Lys Ala Leu Ala Phe Ala Gln Gln Ala Asp Tyr Leu Glu Gln
                485                 490                 495

Asp Leu Ala Met Thr Lys Asp Gly Arg Leu Val Val Ile His Asp His
            500                 505                 510

Phe Leu Asp Gly Leu Thr Asp Val Ala Lys Lys Phe Pro His Arg His
        515                 520                 525

Arg Lys Asp Gly Arg Tyr Tyr Val Ile Asp Phe Thr Leu Lys Glu Ile
    530                 535                 540

Gln Ser Leu Glu Met Thr Glu Asn Phe Glu Thr Lys Asp Gly Lys Gln
545                 550                 555                 560

Ala Gln Val Tyr Pro Asn Arg Phe Pro Leu Trp Lys Ser His Phe Arg

                565                 570                 575

Ile His Thr Phe Glu Asp Glu Ile Glu Phe Ile Gln Gly Leu Glu Lys
            580                 585                 590

Ser Thr Gly Lys Lys Val Gly Ile Tyr Pro Glu Ile Lys Ala Pro Trp
        595                 600                 605

Phe His His Gln Asn Gly Lys Asp Ile Ala Ala Glu Thr Leu Lys Val
    610                 615                 620

Leu Lys Lys Tyr Gly Tyr Asp Lys Lys Thr Asp Met Val Tyr Leu Gln
625                 630                 635                 640

Thr Phe Asp Phe Asn Glu Leu Lys Arg Ile Lys Thr Glu Leu Leu Pro
                645                 650                 655

Gln Met Gly Met Asp Leu Lys Leu Val Gln Leu Ile Ala Tyr Thr Asp
            660                 665                 670

Trp Lys Glu Thr Gln Glu Lys Asp Pro Lys Gly Tyr Trp Val Asn Tyr
        675                 680                 685

Asn Tyr Asp Trp Met Phe Lys Pro Gly Ala Met Ala Glu Val Val Lys
    690                 695                 700

Tyr Ala Asp Gly Val Gly Pro Gly Trp Tyr Met Leu Val Asn Lys Glu
705                 710                 715                 720

Glu Ser Lys Pro Asp Asn Ile Val Tyr Thr Pro Leu Val Lys Glu Leu
                725                 730                 735

Ala Gln Tyr Asn Val Glu Val His Pro Tyr Thr Val Arg Lys Asp Ala
            740                 745                 750

Leu Pro Glu Phe Phe Thr Asp Val Asn Gln Met Tyr Asp Ala Leu Leu
        755                 760                 765

Asn Lys Ser Gly Ala Thr Gly Val Phe Thr Asp Phe Pro Asp Thr Gly
    770                 775                 780

Val Glu Phe Leu Lys Gly Ile Lys
785                 790

<210> 3
<211> 791
<212> PRT
<213> Artificial Sequence

<220>
<223>  HID-HIN47

<400>  3

Met Asp Pro Ser Ser His Ser Ser Asn Met Ala Asn Thr Gln Met Lys
1               5                   10                  15

Ser Asp Lys Ile Ile Ile Ala His Arg Gly Ala Ser Gly Tyr Leu Pro
            20                  25                  30

Glu His Thr Leu Glu Ser Lys Ala Leu Ala Phe Ala Gln Gln Ala Asp
        35                  40                  45

Tyr Leu Glu Gln Asp Leu Ala Met Thr Lys Asp Gly Arg Leu Val Val
    50                  55                  60

Ile His Asp His Phe Leu Asp Gly Leu Thr Asp Val Ala Lys Lys Phe
65                  70                  75                  80

Pro His Arg His Arg Lys Asp Gly Arg Tyr Tyr Val Ile Asp Phe Thr
                85                  90                  95

Leu Lys Glu Ile Gln Ser Leu Glu Met Thr Glu Asn Phe Glu Thr Lys
            100                 105                 110

Asp Gly Lys Gln Ala Gln Val Tyr Pro Asn Arg Phe Pro Leu Trp Lys
        115                 120                 125

Ser His Phe Arg Ile His Thr Phe Glu Asp Glu Ile Glu Phe Ile Gln
    130                 135                 140

Gly Leu Glu Lys Ser Thr Gly Lys Lys Val Gly Ile Tyr Pro Glu Ile
145                 150                 155                 160

Lys Ala Pro Trp Phe His His Gln Asn Gly Lys Asp Ile Ala Ala Glu
                165                 170                 175

Thr Leu Lys Val Leu Lys Lys Tyr Gly Tyr Asp Lys Lys Thr Asp Met
            180                 185                 190

Val Tyr Leu Gln Thr Phe Asp Phe Asn Glu Leu Lys Arg Ile Lys Thr
        195                 200                 205

Glu Leu Leu Pro Gln Met Gly Met Asp Leu Lys Leu Val Gln Leu Ile
    210                 215                 220

Ala Tyr Thr Asp Trp Lys Glu Thr Gln Glu Lys Asp Pro Lys Gly Tyr

225                 230                 235                 240

Trp Val Asn Tyr Asn Tyr Asp Trp Met Phe Lys Pro Gly Ala Met Ala
                245                 250                 255

Glu Val Val Lys Tyr Ala Asp Gly Val Gly Pro Gly Trp Tyr Met Leu
            260                 265                 270

Val Asn Lys Glu Glu Ser Lys Pro Asp Asn Ile Val Tyr Thr Pro Leu
        275                 280                 285

Val Lys Glu Leu Ala Gln Tyr Asn Val Glu Val His Pro Tyr Thr Val
    290                 295                 300

Arg Lys Asp Ala Leu Pro Glu Phe Phe Thr Asp Val Asn Gln Met Tyr
305                 310                 315                 320

Asp Ala Leu Leu Asn Lys Ser Gly Ala Thr Gly Val Phe Thr Asp Phe
                325                 330                 335

Pro Asp Thr Gly Val Glu Phe Leu Lys Gly Ile Lys Gly Gly Ser Gly
            340                 345                 350

Gly Ser Thr Leu Pro Ser Phe Val Ser Glu Gln Asn Ser Leu Ala Pro
        355                 360                 365

Met Leu Glu Lys Val Gln Pro Ala Val Val Thr Leu Ser Val Glu Gly
    370                 375                 380

Lys Ala Lys Val Asp Ser Arg Ser Pro Phe Leu Asp Asp Ile Pro Glu
385                 390                 395                 400

Glu Phe Lys Phe Phe Phe Gly Asp Arg Phe Ala Glu Gln Phe Gly Gly
                405                 410                 415

Arg Gly Glu Ser Lys Arg Asn Phe Arg Gly Leu Gly Ser Gly Val Ile
            420                 425                 430

Ile Asn Ala Ser Lys Gly Tyr Val Leu Thr Asn Asn Ala Val Ile Asp
        435                 440                 445

Glu Ala Asp Lys Ile Thr Val Gln Leu Gln Asp Gly Arg Glu Phe Lys
    450                 455                 460

Ala Lys Leu Val Gly Lys Asp Glu Leu Ser Asp Ile Ala Leu Val Gln
465                 470                 475                 480

Leu Glu Lys Pro Ser Asn Leu Thr Glu Ile Lys Phe Ala Asp Ser Asp
                485                 490                 495

Lys Leu Arg Val Gly Asp Phe Thr Val Ala Ile Gly Asn Pro Phe Gly
            500                 505                 510

Leu Gly Gln Thr Val Thr Ser Gly Ile Val Ser Ala Leu Gly Arg Ser
        515                 520                 525

Thr Gly Ser Asp Ser Gly Thr Tyr Glu Asn Tyr Ile Gln Thr Asp Ala
    530                 535                 540

Ala Val Asn Arg Gly Asn Ser Gly Gly Ala Leu Val Asn Leu Asn Gly
545                 550                 555                 560

Glu Leu Ile Gly Ile Asn Thr Ala Ile Ile Ser Pro Ser Gly Gly Asn
                565                 570                 575

Ala Gly Ile Ala Phe Ala Ile Pro Ser Asn Gln Ala Ser Asn Leu Val
            580                 585                 590

Gln Gln Ile Leu Glu Phe Gly Gln Val Arg Arg Gly Leu Leu Gly Ile
        595                 600                 605

Lys Gly Gly Glu Leu Asn Ala Asp Leu Ala Lys Ala Phe Asn Val Ser
    610                 615                 620

Ala Gln Gln Gly Ala Phe Val Ser Glu Val Leu Pro Lys Ser Ala Ala
625                 630                 635                 640

Glu Lys Ala Gly Leu Lys Ala Gly Asp Ile Ile Thr Ala Met Asn Gly
                645                 650                 655

Gln Lys Ile Ser Ser Phe Ala Glu Ile Arg Ala Lys Ile Ala Thr Thr
            660                 665                 670

Gly Ala Gly Lys Glu Ile Ser Leu Thr Tyr Leu Arg Asp Gly Lys Ser
        675                 680                 685

His Asp Val Lys Met Lys Leu Gln Ala Asp Asp Ser Ser Gln Leu Ser
    690                 695                 700

Ser Lys Thr Glu Leu Pro Ala Leu Asp Gly Ala Thr Leu Lys Asp Tyr
705                 710                 715                 720

Asp Ala Lys Gly Val Lys Gly Ile Glu Ile Thr Lys Ile Gln Pro Asn
                725                 730                 735

Ser Leu Ala Ala Gln Arg Gly Leu Lys Ser Gly Asp Ile Ile Ile Gly
            740                 745                 750

Ile Asn Arg Gln Met Ile Glu Asn Ile Arg Glu Leu Asn Lys Val Leu
        755                 760                 765

Glu Thr Glu Pro Ser Ala Val Ala Leu Asn Ile Leu Arg Gly Asp Ser
    770                 775                 780

Asn Phe Tyr Leu Leu Val Gln
785                 790

<210>  4
<211>  39
<212>  DNA
<213>  Artificial Sequence

<220>
<223>  Primer

<400>  4
ccgatgtagc ctagaaattt cctcataggc ataggaaag                              39

<210>  5
<211>  32
<212>  DNA
<213>  Artificial Sequence

<220>
<223>  Primer

<400>  5
tttctaggct acatcggtta agccgtccag aa                                     32

<210>  6
<211>  33
<212>  DNA
<213>  Artificial Sequence

<220>
<223>  Primer

<400>  6
gaccaaatag gggaaacagg ctcaagtgta tcc                                    33

<210>  7
<211>  32
<212>  DNA
<213>  Artificial Sequence

<220>
<223>  Primer

<400>  7
gtttccccta tttggtctcg aaattttccg tc                                     32

<210>  8
<211>  32
<212>  DNA
<213>  Artificial Sequence

<220>
<223>  Primer

<400>  8
aaaacctagc tccttcctca gatgggaatg ga                                    32


<210>  9
<211>  35
<212>  DNA
<213>  Artificial Sequence

<220>
<223>  Primer

<400>  9
ggaaggagct aggtttttat gcgtttcagt tcatt                                 35


<210>  10
<211>  38
<212>  DNA
<213>  Artificial Sequence

<220>
<223>  Primer

<400>  10
cgaaaggata gtgggtaaac tacaattatg attggatg                              38


<210>  11
<211>  32
<212>  DNA
<213>  Artificial Sequence

<220>
<223>  Primer

<400>  11
tacccactat cctttcggat ctttttcctg gg                                    32


<210>  12
<211>  32
<212>  DNA
<213>  Artificial Sequence

<220>
<223>  Primer

<400>  12
tagagtaccg ggtcagactc cggcacctac ga                                    32


<210>  13

<211>  37
<212>  DNA
<213>  Artificial Sequence

<220>
<223>  Primer

<400>  13
tctgacccgg tactctagcc cagagcggaa actatcc                                37


<210>  14
<211>  32
<212>  DNA
<213>  Artificial Sequence

<220>
<223>  Primer

<400>  14
tgctatctag tcaccgagcg gcggtaatgc gg                                     32


<210>  15
<211>  37
<212>  DNA
<213>  Artificial Sequence

<220>
<223>  Primer

<400>  15
tcggtgacta gatagcagta ttgataccga tcaattc                                37


<210>  16
<211>  32
<212>  DNA
<213>  Artificial Sequence

<220>
<223>  Primer

<400>  16
ttaggcggga attgcgttcg caattcccag ta                                     32


<210>  17
<211>  34
<212>  DNA
<213>  Artificial Sequence

<220>
<223>  Primer

<400>  17
acgcaattcc cgcctaaccg ccgctcggtg agat                                   34


<210>  18
<211>  32
<212>  DNA
<213>  Artificial Sequence

<220>
<223>  Primer

<400>  18
aatgcgggat aggcgttcgc aattcccagt aa                                    32


<210>  19
<211>  32
<212>  DNA
<213>  Artificial Sequence

<220>
<223>  Primer

<400>  19
aacgcctatc ccgcattacc gccgctcggt ga                                    32


<210>  20
<211>  35
<212>  DNA
<213>  Artificial Sequence

<220>
<223>  Primer

<400>  20
gctgaaattt aggcaaaaat tgctacaact ggtgc                                 35


<210>  21
<211>  33
<212>  DNA
<213>  Artificial Sequence

<220>
<223>  Primer

<400>  21
tttgcctaaa tttcagcaaa cgaagagatt ttc                                   33


<210>  22
<211>  32
<212>  DNA
<213>  Artificial Sequence

<220>
<223>  Primer

<400>  22
tcgcgcatag attgctacaa ctggtgcagg ca                                    32


<210>  23
<211>  32
<212>  DNA
<213>  Artificial Sequence

<220>
<223>  Primer

<400>  23
tagcaatcta tgcgcgaatt tcagcaaacg aa                                    32

<210>  24
<211>  39
<212>  DNA
<213>  Artificial Sequence

<220>
<223>  Primer

<400>  24
ccgatgtagc ctagaaattt cctcataggc ataggaaag                             39

<210>  25
<211>  32
<212>  DNA
<213>  Artificial Sequence

<220>
<223>  Primer

<400>  25
tttctaggct acatcggtta agccgtccag aa                                    32

<210>  26
<211>  33
<212>  DNA
<213>  Artificial Sequence

<220>
<223>  Primer

<400>  26
gaccaaatag gggaaacagg ctcaagtgta tcc                                   33

<210>  27
<211>  32
<212>  DNA
<213>  Artificial Sequence

<220>
<223>  Primer

<400>  27
gtttccccta tttggtctcg aaattttccg tc                                    32

<210>  28
<211>  32
<212>  DNA
<213>  Artificial Sequence

<220>
<223>  Primer

<400>  28
aaaacctagc tccttcctca gatgggaatg ga                                    32

<210>  29
<211>  35
<212>  DNA
<213>  Artificial Sequence

<220>
<223>  Primer

<400>  29
ggaaggagct aggtttttat gcgtttcagt tcatt                                35


<210>  30
<211>  38
<212>  DNA
<213>  Artificial Sequence

<220>
<223>  Primer

<400>  30
cgaaaggata gtgggtaaac tacaattatg attggatg                             38


<210>  31
<211>  32
<212>  DNA
<213>  Artificial Sequence

<220>
<223>  Primer

<400>  31
tacccactat cctttcggat ctttttcctg gg                                   32


<210>  32
<211>  32
<212>  DNA
<213>  Artificial Sequence

<220>
<223>  Primer

<400>  32
tagagtaccg ggtcagactc cggcacctac ga                                   32


<210>  33
<211>  37
<212>  DNA
<213>  Artificial Sequence

<220>
<223>  Primer

<400>  33
tctgacccgg tactctagcc cagagcggaa actatcc                              37


<210>  34

<211>  32
<212>  DNA
<213>  Artificial Sequence

<220>
<223>  Primer

<400>  34
tgctatctag tcaccgagcg gcggtaatgc gg                                    32


<210>  35
<211>  37
<212>  DNA
<213>  Artificial Sequence

<220>
<223>  Primer

<400>  35
tcggtgacta gatagcagta ttgataccga tcaattc                               37


<210>  36
<211>  32
<212>  DNA
<213>  Artificial Sequence

<220>
<223>  Primer

<400>  36
ttaggcggga attgcgttcg caattcccag ta                                    32


<210>  37
<211>  34
<212>  DNA
<213>  Artificial Sequence

<220>
<223>  Primer

<400>  37
acgcaattcc cgcctaaccg ccgctcggtg agat                                  34


<210>  38
<211>  32
<212>  DNA
<213>  Artificial Sequence

<220>
<223>  Primer

<400>  38
aatgcgggat aggcgttcgc aattcccagt aa                                    32


<210>  39
<211>  32
<212>  DNA
<213>  Artificial Sequence

<220>
<223>  Primer

<400>  39
aacgcctatc ccgcattacc gccgctcggt ga                                    32


<210>  40
<211>  35
<212>  DNA
<213>  Artificial Sequence

<220>
<223>  Primer

<400>  40
gctgaaattt aggcaaaaat tgctacaact ggtgc                                 35


<210>  41
<211>  33
<212>  DNA
<213>  Artificial Sequence

<220>
<223>  Primer

<400>  41
tttgcctaaa tttcagcaaa cgaagagatt ttc                                   33


<210>  42
<211>  32
<212>  DNA
<213>  Artificial Sequence

<220>
<223>  Primer

<400>  42
tcgcgcatag attgctacaa ctggtgcagg ca                                    32


<210>  43
<211>  32
<212>  DNA
<213>  Artificial Sequence

<220>
<223>  Primer

<400>  43
tagcaatcta tgcgcgaatt tcagcaaacg aa                                    32